# EUROPÄISCHE PATENTSCHRIFT

(11) **EP 3 558 931 B1**
(45) Veröffentlichungstag und Bekanntmachung des Hinweises auf die Patenterteilung: **20.04.2022**
(21) Anmeldenummer: 17818552.6
(22) Anmeldetag: 18.12.2017
(51) Int. Cl.: C07C 227/28, C07C 227/40, C12P 13/00

(54) **VERFAHREN ZUR HERSTELLUNG VON AMINOBENZOESÄURE ODER EINES AMINOBENZOESÄUREFOLGEPRODUKTS**
PROCESS FOR THE PREPARATION OF AMINOBENZOIC ACID OR AN AMINOBENZOIC ACID DERIVATIVE
PROCÉDÉ DE FABRICATION D'ACIDE AMINOBENZOÏQUE OU D'UN DÉRIVÉ D'ACIDE AMINOBENZOÏQUE

(30) Priorität: 20.12.2016 EP 16205348; 27.11.2017 EP 17203706
(43) Veröffentlichungstag der Anmeldung: 30.10.2019
(73) Patentinhaber: Covestro Intellectual Property GmbH & Co. KG, 51373 Leverkusen (DE)
(72) Erfinder: JÄGER, Gernot, 50733 Köln (DE); GÖRTZ, Ivonne, 51063 Köln (DE); LOLLI, Giulio, 51069 Köln (DE); MOUSSA, Amgad Salah, 79713 Bad Säckingen (DE); HAMEDINGER, Thomas, 51375 Leverkusen (DE)
(74) Vertreter: Levpat
(86) Internationale Anmeldenummer: PCT/EP2017/083374
(87) Internationale Veröffentlichungsnummer: WO 2018/114841

(56) Entgegenhaltungen:
- WO-A1-2015/124687
- CN-A- 1 923 799
- FR-A- 1 539 529
- US-A1- 2014 371 418
- COONEY AND J WIJAYA D O: "Effect of pH and added salts on the adsorption of ionizable organic species onto activated carbon from aqueous solution", PROCEEDINGS OF THE SECOND ENGINEERING FOUNDATION CONFERENCE ON FUNDAMENTALS OF ADSORPTION,1987) - PROCEEDINGS OF THE SECOND ENGINEERING FOUNDATION CONFERENCE ON FUNDAMENTALS OF ADSORPTION, MAY 4 - 9, 1986, SANTA BARBARA, CALIFORNIA / ATHANASIOS I. LI, 1. Januar 1986 (1986-01-01), Seiten 185-194, XP008184783, ISBN: 0-8169-0398-0 in der Anmeldung erwähnt

## Beschreibung

Die vorliegende Erfindung betrifft ein Verfahren zur Herstellung von Aminobenzoesäure oder eines Aminobenzoesäurefolgeprodukts gemäß Patentanspruch 1.

Aminobenzoesäure ist ein wirtschaftlich bedeutsames Produkt, das entweder als solches oder als Zwischenprodukt in der Herstellung anderer, von der Aminobenzoesäure durch weitere chemische Umsetzung(en) abgeleiteter Verbindungen *(Aminobenzoesäurefolgeprodukte,* auch als *Aminobenzoesäurederivate* bezeichnet), Anwendung findet. Aminobenzoesäure findet beispielsweise Anwendung in der Herstellung von Farbstoffen, Geruchsstoffen oder Pharmazeutika (Wiklund, Current Organic Synthesis, 2006, 3, 379-402). Ein Beispiel für eine wichtige Folgereaktion von Aminobenzoesäure zur Herstellung eines anderen Produkts ist die Decarboxylierung zu Anilin, das seinerseits insbesondere als Intermediat in der Herstellung von Isocyanaten von Bedeutung ist.

Aminobenzoesäure kann chemisch oder fermentativ gewonnen werden:
Die *chemische* Herstellung von Aminobenzoesäure ist in der Literatur beschrieben. Eine geeignete Syntheseroute (mit Ausbeuten > 98 %) ist beispielsweise die Reaktion von Phthalimid mit Natriumhypochlorit. Phthalimid kann seinerseits aus Phthalsäureanhydrid und Ammoniak gewonnen werden. Der ganze Prozess ist wohlbekannt und wird z. B. in Lorz et al., Phthalic Acid and Derivatives in Ullmann's Encyclopedia of Industrial Chemistry, Band 27, S. 140 - 141, Weinheim, Wiley-VCH, beschrieben. Ein industrieller Prozess ist ebenfalls in der Patentliteratur beschrieben; siehe z. B. DE 29 02 978 A1 und EP 0 004 635 A2.

CN 103408177 B befasst sich mit der Behandlung und Rezyklierung von Abwasser aus der chemischen Anthranilsäureproduktion. Als chemischer Syntheseweg wird die Umsetzung von Phthalsäureanhydrid mit Harnstoff im Überschuss unter Bildung von Phthalimid, dessen Zersetzung mit Natriumhypochlorid unter alkalischen Bedingungen gefolgt von Ansäuern des so gebildeten Natriumsalzes mit Salzsäure beschrieben. Die so gebildete Anthranilsäure wird durch Zentrifugation abgetrennt und gewaschen. Die bei der Zentrifugation anfallende wässrige Phase und das Waschwasser stellen wässrige Abwässer dar, die hauptsächlich nicht abreagiertes Phthalimid, Anthranilsäure und Natriumchlorid enthalten. Die Reinigung solcher Abwässer ist Gegenstand dieser Patentschrift.

Diese Reinigung umfasst in einem Schritt a) die Einstellung des pH-Werts des Abwassers auf 8, gefolgt von Durchleiten des so alkalisch gestellten Abwassers durch eine Adsorptionskolonne, die mit einem chemisch modifizierten hoch quervernetztem Polystyrol-Divinyl-Benzol-Adsorptions-Harz befüllt ist. Das in diesem Reinigungsschritt a) erhaltene Abwasser wird sodann in einem Schritt b) mit 1 bis 2%iger Salzsäure auf einen pH-Wert von 5 eingestellt und durch eine Adsorptionskolonne, die mit einem chemisch modifizierten hoch quervernetztem Polystyrol-Divinyl-Benzol-Adsorptions-Harz befüllt ist, geleitet.

Wenn die Adsorptionskolonne aus Schritt a) nahezu gesättigt ist, wird diese in einem Schritt c) durch eine parallel geschaltete Adsorptionskolonne der gleichen Art ersetzt, und die außer Betrieb genommene Kolonne wird regeneriert. Zu diesem Zweck wird die Adsorptionskolonne zunächst mit 4 bis 6%iger Salzsäure und anschließend mit 1 bis 2%iger Natronlauge durchspült. Das dabei anfallende Waschwasser wird mit der ursprünglichen Abwasserlösung vereint.

Wenn die Adsorptionskolonne aus Schritt b) nahezu gesättigt ist, wird diese in einem Schritt d) ebenfalls durch eine parallel geschaltete Adsorptionskolonne der gleichen Art ersetzt, und die außer Betrieb genommene Kolonne wird regeneriert. Zu diesem Zweck wird die Adsorptionskolonne zunächst mit 4 bis 6%iger Natronlauge und anschließend mit 1 bis 2%iger Salzsäure durchspült. Das dabei anfallende Waschwasser wird mit der ursprünglichen Abwasserlösung vereint.

CN 1923799 A befasst sich mit einem Verfahren zur Rückgewinnung von ortho-Aminobenzoesäure aus Mutterlauge oder Abwasser, ohne die Herkunft derselben näher zu spezifizieren. Die Rückgewinnung erfolgt durch Adsorption an einem schwach basischem oder einem makroporösem Harz. Nach erfolgter Adsorption wird die Adsorptionskolonne mit 2 bis 6%iger Natronlauge gespült, wobei eine wässrige Lösung von Natrium-ortho-Aminobenzoat erhalten wird.

FR 1.539.529 befasst sich mit der Gewinnung hochreiner aromatischer Carbonsäuren durch Sublimation. Ein Beispiel unter vielen für eine geeignete Carbonsäure ist meta-Aminobenzoesäure.

Die *fermentative* Herstellung von Aminobenzoesäure, die Gegenstand der vorliegenden Erfindung ist, ist ebenfalls in der Literatur beschrieben. Für die fermentative Herstellung von Aminobenzoesäure sei beispielhaft auf Balderas-Hemandez, V. E. et al., "Metabolic engineering for improving anthranilate synthesis from glucose in Escherichia coli", Microb. Cell. Fact. 2009, 8, 19 (doi: 10.118611475-2859-8-19) verwiesen. Auch in der Patentliteratur finden sich hierzu Veröffentlichungen. So beansprucht die Anmeldung US 2014/0371418 A1 rekombinante mikrobakterielle Wirtszellen, die in der Lage sind, eine fermentierbare Kohlenstoff-haltige Verbindung auf biologische Art in para-Aminobenzoesäure zu überführen. Weitere Beispiele für fermentative Prozesse sind in den Anmeldungen WO 2015/124686 A1 und WO 2015/124687 A1 sowie in der darin jeweils zitierten Literatur zu finden. Fermentationsprozesse laufen in der Regel in einer wässrigen Umgebung ab und liefern im Falle der Herstellung von Aminobenzoesäure im Allgemeinen wässrige Lösungen (Fermentationsbrühen) mit einem Massengehalt an Aminobenzoesäure im Bereich von 10,0 g/L bis 100 g/L.

WO 2015/124686 A1 und WO 2015/124687 A1 befassen sich neben der fermentativen Herstellung von Aminobenzoesäure auch mit deren anschließender Decarboxylierung zu Anilin. Die Isolierung des Ausgangsmaterials Aminobenzoesäure aus der Fermentationsbrühe erfolgt gemäß der Lehre dieser Schriften insbesondere durch Kristallisation und Filtration. WO 2015/124687 A1 offenbart weiterhin eine bevorzugte Ausführungsform, in welcher die bei der Kristallisation erhaltene Mutterlauge (die eine der Löslichkeit von Aminobenzoesäure unter den jeweiligen Bedingungen entsprechende Rest-Konzentration an Aminobenzoesäure aufweist) aufgearbeitet wird, um weitere Aminobenzoesäure zu gewinnen. Dies geschieht durch eine Sequenz aus einem Adsorptions- und einem Desorptionsschritt. Das erhaltene an Aminobenzoesäure angereicherte Desorbat wird in die Kristallisation zurückgeführt. Auf diese Weise werden Ausbeuteverluste verringert. Die Adsorption erfolgt dabei an Zeolithen oder Aktivkohle, die Desorption mit Wasser eines pH-Werts im Bereich von 5 bis 10 oder alternativ mit organischen Lösungsmitteln, insbesondere 1-Dodecanol. Die Desorption bei den genannten pH-Werten hat jedoch bei der Rückführung in die Kristallisation den Nachteil, dass - insbesondere im oberen Teil des genannten pH-Bereiches - die zur Kristallisation erforderliche Menge an Säure ansteigt. Die Verwendung eines systemfremden organischen Lösungsmittels ist grundsätzlich nachteilig, da mit zusätzlichen Kosten und erhöhtem Entsorgungsaufwand verbunden.

D. O. Cooney und J. Wijaya beschreiben in Effect of pH and Added Salts on the Adsorption of Ionizable Organic Species onto Activated Carbon from Aqueous Solutions (veröffentlicht in Proceedings of the Second Engineering Foundation Conference on Fundamentals of Adsorption, May 4-9 1986, Santa Barbara, Carlifornia) auf den Seiten 185 bis 194 den Einfluss der Adsorption von ortho-Aminobenzoesäure auf Aktivkohle bei verschiedenen pH-Werten mit und ohne Zusatz von NaCl. Das Adsorptionsmaximum liegt beim isoelektrischen Punkt. Die Adsorption bei hohen pH-Werten kann durch Zugabe von Natriumchlorid gesteigert werden. Für die Versuche wurden Laborchemikalien eingesetzt. Die Besonderheiten fermentativ hergestellter ortho-Aminobenzoesäure sind nicht Gegenstand dieser Veröffentlichung; insbesondere wird der Einsatz einer Fermentationsbrühe als Ausgangsmaterial für die Adsorption nicht offenbart.

Weitere Verbesserungen in der Herstellung von fermentativ hergestellter Aminobenzoesäure bzw. Aminobenzoesäurefolgeprodukten wären daher wünschenswert. Insbesondere wäre es wünschenswert, die in den bekannten fermentativen Herstellverfahren zunächst in gelöster Form anfallende Aminobenzoesäure mit möglichst geringen Ausbeuteverlusten zu isolieren. Dabei sollte das Verfahren möglichst einfach sein und ohne den Einsatz systemfremder Lösungsmittel (wie 1-Dodecanol) ausgestaltet werden können, um die Wirtschaftlichkeit des Verfahrens zu erhöhen und so dessen Einsatz im großtechnischen Produktionsmaßstab attraktiver zu machen. Weiterhin wäre es wünschenswert, die Isolierung der Aminobenzoesäure (und damit die Ausbeute) zu verbessern, ohne dadurch an anderer Stelle des Gesamtverfahrens Nachteile hervorzurufen (wie dies beispielsweise bei der in WO 2015/124687 A1 geschilderten Desorption im pH-Bereich 5 bis 10 der Fall ist).

Dem vorstehend Gesagten Rechnung tragend ist ein Gegenstand der vorliegenden Erfindung ein **Verfahren zur Herstellung von Aminobenzoesäure oder eines Aminobenzoesäurefolgeprodukts,** umfassend die folgenden Schritte:
(I) Bereitstellen einer wässrigen Lösung von Aminobenzoesäure unter Anwendung eines fermentativen Verfahrens, wobei Schritt (I) umfasst:
   (I-1) Fermentation eines Rohstoffes, der wenigstens
      - eine fermentierbare Kohlenstoff-haltige Verbindung, bevorzugt ausgewählt aus der Gruppe bestehend aus Stärkehydrolysat, Zuckerrohrsaft, Zuckerrübensaft und Hydrolysaten aus lignocellulosehaltigen Rohstoffen, und
      - eine Stickstoff-haltige Verbindung, bevorzugt ausgewählt aus der Gruppe bestehend aus Ammonikgas, Ammoniakwasser, Ammoniumsalzen (insbesondere Ammoniumsulfat und Ammoniumchlorid) und Harnstoff, umfasst,
         in einem Fermentationsreaktor unter Verwendung von Mikroorganismen unter Erhalt einer Fermentationsbrühe;
         woran sich optional
   (I-2) eine Aufarbeitung umfassend die Aufarbeitungsschritte:
      (α) Abtrennung des Mikroorganismus aus der in Schritt (I-1) erhaltenen Fermentationsbrühe und/oder
      (β) Entfärbung der in Schritt (I-1) erhaltenen Fermentationsbrühe oder, bei Durchführung von Schritt (α), der in Schritt (α) erhaltenen, an Mikroorganismen abgereicherten, Fermentationsbrühe,
         anschließt;
   (I-3) Einführen der in Schritt (I-1) oder, sofern durchgeführt, in Schritt (I-2), erhaltenen Fermentationsbrühe in einen Reaktor, in dem durch Vermischen mit einer sauren wässrigen Lösung Aminobenzoesäure abgeschieden wird, wobei bevorzugt der pH-Wert der resultierenden Mischung auf einen Wert im Bereich von 3,0 bis 4,7, bevorzugt im Bereich von 3,2 bis 3,7, besonders bevorzugt im Bereich von 3,4 bis 3,6, eingestellt wird;
   (I-4) Abtrennung der in Schritt (I-3) abgeschiedenen Aminobenzoesäure, bevorzugt durch Filtration, wobei eine an Aminobenzoesäure abgereicherte Mutterlauge erhalten wird;
(II) Behandlung der in Schritt (I) bereitgestellten wässrigen Lösung von Aminobenzoesäure, nämlich der in Schritt (I-4) erhaltenen, an Aminobenzoesäure abgereicherten Mutterlauge, mit einem Adsorptionsmittel, vorzugsweise Aktivkohle, wobei das Adsorptionsmittel mit Aminobenzoesäure beladen wird und ein an Aminobenzoesäure abgereichertes Adsorbat erhalten wird;
(III) Behandlung des mit Aminobenzoesäure beladenen Adsorptionsmittels aus Schritt (II) mit einem wässrigen Desorptionsmittel eines pH-Werts im Bereich von -0,8 bis 3,0, bevorzugt -0,5 bis 3,0, besonders bevorzugt 0,1 bis 3,0, ganz bevorzugt 0,5 bis 2,5, außerordentlich ganz besonders bevorzugt 1,0 bis 2,0, wobei ein an Aminobenzoesäure angereichertes Desorbat und ein an Aminobenzoesäure abgereichertes Adsorptionsmittel erhalten werden;
(IV) Gewinnung der Aminobenzoesäure aus dem in Schritt (III) erhaltenen Desorbat durch Verwendung des in Schritt (III) erhaltenen an Aminobenzoesäure angereicherten Desorbats als Bestandteil der in Schritt (I-3) eingesetzten sauren wässrigen Lösung;
(V) optionale weitere Umsetzung der in Schritt (IV) gewonnenen Aminobenzoesäure zu einem Aminobenzosäurefolgeprodukt, wobei Schritt (V) insbesondere eine der folgenden Umsetzungen umfasst:
   (V-1) Decarboxylierung der Aminobenzoesäure zu Anilin;
   (V-2) Decarboxylierung der Aminobenzoesäure zu Anilin, gefolgt von säurekatalysierter Reaktion des Anilins mit Formaldehyd unter Bildung von Di- und Polyaminen der Diphenylmethanreihe;
   (V-3) Decarboxylierung der Aminobenzoesäure zu Anilin, gefolgt von säurekatalysierter Reaktion des Anilins mit Formaldehyd unter Bildung von Di- und Polyaminen der Diphenylmethanreihe, gefolgt von Umsetzung mit Phosgen unter Bildung von Di- und Polyisocyanaten der Diphenylmethanreihe;
   (V-4) Decarboxylierung der Aminobenzoesäure zu Anilin, gefolgt von Umsetzung des Anilins zu einer Azoverbindung;
   (V-5) Umsetzung der Aminobenzoesäure zu einem Amid;
   (V-6) Umsetzung der Aminobenzoesäure zu leitenden Polymeren wie insbesondere Polyanthranilsäure.

Der Begriff "*Aminobenzoesäurefolgeprodukt*" bezeichnet im Rahmen der vorliegenden Erfindung ein Produkt, das durch weitere chemische Umwandlung von Aminobenzoesäure erhalten wird.

Vollkommen überraschend wurde gefunden, dass die Durchführung der Desorption (Schritt III) in einem stark sauren wässrigen Medium im Gegensatz zu dem, was der Fachmann aus der Lehre der WO 2015/124687 A1 ableiten würde, möglich und sogar vorteilhaft ist. So wird die Gewinnung der Aminobenzoesäure aus dem Desorbat vereinfacht, und zwar insbesondere dann, wenn dieser Schritt eine Kristallisation umfasst.

**Ausführungsformen** der Erfindung werden nachfolgend näher beschrieben. Dabei können verschiedene Ausführungsformen beliebig miteinander kombiniert werden, sofern sich für den Fachmann aus dem Gesamtzusammenhang nicht das Gegenteil ergibt.

Es folgt zunächst eine Kurzzusammenfassung verschiedener möglicher Ausführungsformen.

In einer **ersten Ausführungsform** der Erfindung umfasst Schritt (I) weiterhin:
(I-5) eine weitere Reinigung der in Schritt (I-4) abgetrennten Aminobenzoesäure, bevorzugt durch Waschen mit Wasser.

In einer **zweiten Ausführungsform** der Erfindung, die mit allen anderen Ausführungsformen der Erfindung kombiniert werden kann, wird das in Schritt (III) erhaltene, an Aminobenzoesäure angereicherte Desorbat als Bestandteil der in Schritt (I-3) eingesetzten sauren wässrigen Lösung gemeinsam mit einer Säure ausgewählt aus der Gruppe bestehend aus Salzsäure, Schwefelsäure, Phosphorsäure und Mischungen derselben verwendet.

In einer **dritten Ausführungsform** der Erfindung, die eine besondere Ausgestaltung der zweiten Ausführungsform ist, wird das in Schritt (III) erhaltene, an Aminobenzoesäure angereicherte Desorbat als Bestandteil der in Schritt (I-3) eingesetzten sauren wässrigen Lösung gemeinsam mit Salzsäure einer Konzentration von 15 Massen-% bis 37 Massen-% verwendet.

In einer **vierten Ausführungsform,** die mit allen anderen Ausführungsformen der Erfindung kombiniert werden kann, wird der pH-Wert des wässrigen Desorptionsmittels während der Durchführung des Schrittes (III) konstant gehalten.

In einer **fünften Ausführungsform,** die mit Ausnahme der dritten Ausführungsform mit allen anderen Ausführungsformen der Erfindung kombiniert werden kann, durchläuft der pH-Wert des wässrigen Desorptionsmittels während der Durchführung des Schrittes (III) einen Gradienten.

In einer **sechsten Ausführungsform,** die mit allen anderen Ausführungsformen der Erfindung kombiniert werden kann, wird in Schritt (I) eine wässrige Lösung des ortho-Isomers von Aminobenzoat und/oder von Aminobenzoesäure bereitgestellt.

In einer **siebten Ausführungsform,** die mit allen anderen Ausführungsformen der Erfindung kombiniert werden kann, umfassen die in Schritt (I-1) eingesetzten Mikroorganismen eine Art ausgewählt aus der Gruppe bestehend aus *Escherichia coli, Pseudomonas putida, Corynebacterium glutamicum, Ashbya gossypii, Pichia pastoris, Hansenula polymorpha, Yarrowia lipolytica, Zygosaccharomyces bailii und Saccharomyces cerevisiae* und bestehen bevorzugt nur aus Vertretern genau einer dieser Arten, wobei *Corynebacterium glutamicum* ATTC 13032 ganz besonders bevorzugt ist.

Die zuvor kurz geschilderten Ausführungsformen und weitere mögliche Ausgestaltungen der Erfindung werden im Folgenden näher erläutert:
In **Schritt (I)** des erfindungsgemäßen Verfahrens wird eine wässrige Lösung von Aminobenzoesäure unter Anwendung eines fermentativen Verfahrens bereitgestellt **(Fermentationsschritt).**

Aminobenzoesäure kommt in drei *isomeren Formen* (ortho-, meta- und para-Aminobenzoesäure) vor. Grundsätzlich kann das erfindungsgemäße Verfahren auf alle drei Isomere, entweder in isomerenreiner Form oder als Mischungen verschiedener Isomere, angewandt werden. Für alle Ausführungsformen der vorliegenden Erfindung gilt, dass die in Schritt (I) bereitzustellende Aminobenzoesäure vorzugsweise das ortho-Isomer umfasst. Besonders bevorzugt umfasst die in Schritt (I) bereitzustellende Aminobenzoesäure mindestens 50,0 mol-%, ganz besonders bevorzugt mindestens 90,0 mol-%, bezogen auf die Gesamtstoffmenge aller vorhandenen Isomere an Aminobenzoesäure, des ortho-Isomers. Außerordentlich ganz besonders bevorzugt besteht die in Schritt (I) bereitzustellende Aminobenzoesäure aus dem ortho-Isomer in isomerenreiner (d. h. Isomerenreinheit > 99,0 mol-%) Form.

Die in Schritt (I) zu bereitzustellende Aminobenzoesäure wird erfindungsgemäß fermentativ hergestellt. Je nachdem, bei welchem pH-Wert die Fermentation durchgeführt wird, fällt Aminobenzoesäure in Schritt (I) nicht in der elektroneutralen Form, sondern z. B. als Aminobenzoat an (für die Art des gebildeten Isomers ist dies jedoch unerheblich). Im Rahmen dieser Erfindung wird im Zusammenhang mit Schritt (I) aus Gründen der sprachlichen Vereinfachung regelmäßig von Aminobenzoesäure gesprochen, was so zu verstehen ist, dass die kationische [d. h. diprotonierte], anionische [d. h. deprotonierte] und neutrale [d. h. *elektro*neutrale] Form von Aminobenzoesäure mit umfasst sind, sofern sich aus den Randbedingungen nichts anderes ergibt.

Der Begriff "Aminobenzoesäure in der neutralen Form" (Aminobenzoesäure "im engeren Sinne") umfasst dabei selbstverständlich nicht nur die Struktur H₂N-(C₆H₄)-COOH, sondern auch das sog. "Zwitterion" H₃N⁺-(C₆H₄)-COO⁻, als welches (elektro-)neutrale Aminobenzoesäure vorliegen kann.

Erfindungsgemäß umfasst Schritt (I) den folgenden Schritt (I-1):
(I-1) Fermentation eines Rohstoffes, der wenigstens
- eine fermentierbare Kohlenstoff-haltige Verbindung, bevorzugt ausgewählt aus der Gruppe bestehend aus Stärkehydrolysat, Zuckerrohrsaft, Zuckerrübensaft und Hydrolysaten aus lignocellulosehaltigen Rohstoffen, und
- eine Stickstoff-haltige Verbindung, bevorzugt ausgewählt aus der Gruppe bestehend aus Ammonikgas, Ammoniakwasser, Ammoniumsalzen (insbesondere Ammoniumsulfat und Ammoniumchlorid) und Harnstoff, umfasst,
   in einem Fermentationsreaktor unter Verwendung von Mikroorganismen unter Erhalt einer Fermentationsbrühe.

Schritt (I-1) des erfindungsgemäßen Verfahrens kann nach einer beliebigen aus dem Stand der Technik bekannten Verfahrensweise durchgeführt werden.

Bevorzugte Mikroorganismen für die Durchführung von Schritt (I-1) sind **Bakterien** oder **Pilze,** und zwar insbesondere **Hefen.** Besonders bevorzugt sind dabei Mikroorganismen einer Art ausgewählt aus der Gruppe bestehend aus *Escherichia coli, Pseudomonas putida, Corynebacterium glutamicum, Ashbya gossypii, Pichia pastoris, Hansenula polymorpha, Yarrowia lipolytica, Zygosaccharomyces bailii und Saccharomyces cerevisiae.* Ganz besonders bevorzugt bestehen die in Schritt (I-1) eingesetzten Mikroorgansimen nur aus Vertretern genau einer dieser Arten, wobei *Corynebacterium glutamicum* ATTC 13032 außerordentlich ganz besonders bevorzugt ist. Der in der Fermentation einzuhaltende pH-Wert richtet sich nach dem eingesetzten Mikroorganismus. Mikroorganismen wie *Corynebacterium glutamicum, Pseudomonas putida* oder *Escherichia coli* werden bevorzugt bei neutralen pH-Werten (d. h. bei einem pH-Wert im Bereich von 6,0 bis 8,0) kultiviert. Mikroorganismen wie *Saccharomyces cerevisiae* werden hingegen bevorzugt im sauren Milieu kultiviert (d. h. bei einem pH-Wert im Bereich von 4,0 bis 5,0).

In jedem Fall wird der Mikroorgansimus aus Schritt (I-1) bevorzugt so ausgewählt, dass in der Fermentation das ortho-Isomer von Aminobenzoesäure gebildet wird.

In einer bevorzugten Ausgestaltung der Erfindung werden **Bakterien als Mikroorganismen** eingesetzt. Dabei wird insbesondere auf die Patentanmeldungen WO 2015/124686 A1 und WO 2015/124687 A1 verwiesen, in denen eine erfindungsgemäß einsetzbare Fermentation unter Einsatz von Bakterien beschrieben ist (siehe beispielsweise WO 2015/124687 A1, (i) Seite 15, Zeile 8 bis Seite 16, Zeile 30, (ii) Beispiel 1 (Seite 29, Zeile 4 bis 26), (iii) Beispiel 3 (vor allem Seite 34, Zeile 10 bis 18), (iv) Beispiel 4 (vor allem Seite 55, Zeile 9 bis 31). Insbesondere kommen Bakterien zum Einsatz, die in der Lage sind, eine fermentierbare Kohlenstoff-haltige Verbindung in Gegenwart einer geeigneten Stickstoffquelle in Aminobenzoesäure umzuwandeln, ohne dass die so gebildete Aminobenzoesäure in zellinternen biochemischen Prozessen gleich wieder verbraucht wird, sodass sich Aminobenzoesäure in der Zelle anreichert und schließlich in die Fermentationsbrühe übergeht.

In einer anderen bevorzugten Ausgestaltung der Erfindung werden **Hefen als Mikroorganismen** eingesetzt. Dabei wird insbesondere auf die internationale Patentanmeldung WO 2017/102853 A1 verwiesen. Insbesondere kommen Hefezellen zum Einsatz, die in der Lage sind, eine fermentierbare Kohlenstoff-haltige Verbindung in Gegenwart einer geeigneten Stickstoffquelle in Aminobenzoesäure umzuwandeln, ohne dass die so gebildete Aminobenzoesäure in zellinternen biochemischen Prozessen gleich wieder verbraucht wird, sodass sich Aminobenzoesäure in der Zelle anreichert und schließlich in die Fermentationsbrühe übergeht.

Um ein derartiges Bakterium oder eine derartige Hefe zu erhalten, stehen grundsätzlich zwei Wege zur Verfügung, die in bevorzugter Ausgestaltung auch kombiniert werden können:
(i) Die enzymatischen Reaktionen im Aminobenzoesäure-Stoffwechselweg der Bakterienzelle oder Hefezelle können so erhöht werden, dass Aminobenzoesäure schneller produziert als verbraucht wird.
(ii) Die Folgereaktionen, durch welche Aminobenzoesäure in weitere Metaboliten oder Produkte (z. B. Tryptophan) überführt wird, können reduziert bzw. ausgeschaltet werden, sodass sogar die Geschwindigkeit der Aminobenzoesäure-Bildung in Wildtyp-Stämmen ausreichend ist, um zu einer Anreicherung von Aminobenzoesäure in der Zelle zu führen.

Verfahren zur Gewinnung von Bakterien oder Hefezellen mit den zuvor genannten Eigenschaften sind im Stand der Technik bekannt. Geeignete Bakterien oder Hefezellen können beispielsweise durch Screening nach Mutanten, welche Aminobenzoesäure in das umgebende Medium abgeben, identifiziert werden. Die zielgerichtete Modifikation von Schlüsselenzymen mittels gentechnischer Verfahren ist jedoch bevorzugt. Mit üblichen gentechnischen Methoden können Genexpression und Enzymaktivität nach Belieben verstärkt, verringert oder sogar ganz unterbunden werden. Es resultieren rekombinante Stämme.

Besonders bevorzugt enthalten die Bakterien oder Hefezellen, die in der Lage sind, eine fermentierbare Kohlenstoff-haltige Verbindung in Gegenwart einer Stickstoff-haltigen Verbindung zu Aminobenzoesäure umzusetzen, eine Modifizierung der Anthranilat-Phosphoribosyltransferase-Aktivität, welche besagte Enzymaktivität herabsetzt. Durch diese Modifizierung wird die Umwandlung von ortho-Aminobenzoat zu N-(5-Phospho-D-Ribosyl)-Anthranilat reduziert oder komplett unterbunden. Hierdurch wird eine Anreicherung von Aminobenzoesäure in der Zelle bewirkt. Die Bezeichnung "Anthranilat-Phosphoribosyltransferase-Aktivität" bezieht dabei sich auf eine Enzymaktivität, durch welche die Umsetzung von ortho-Aminobenzoat zu N-(5-Phospho-D-Ribosyl)-Anthranilat katalysiert wird.

In Hefen wird die Anthranilat-Phosphoribosyltransferase-Aktivität durch das native Gen TRP4 (YDR354W) genetisch kodiert. In dem Bakterium *Corynebacterium glutamicum* wird die Anthranilat-Phosphoribosyltransferase-Aktivität durch das *trpD* Gen (cg3361, Cgl3032, NCgl2929) kodiert. Im Falle von *Pseudomonas putida* erfolgt die Kodierung über das *trpD* Gen (PP_0421) innerhalb des *trpDC* Operons.

Die beschriebene Herabsetzung der Anthranilat-Phosphoribosyltransferase-Aktivität kann prinzipiell auf drei Wegen erreicht werden:
(i) Die Regulierung der Expression des Gens für die Anthranilat-Phosphoribosyltransferase-Aktivität kann so modifiziert werden, dass die Transkription des Gens oder anschließende Translation verringert oder unterbunden wird.
(ii) Die Nukleinsäuresequenz des Gens für Anthranilat-Phosphoribosyltransferase-Aktivität kann so modifiziert werden, dass das Enzym, welches durch das modifizierte Gen kodiert wird, eine geringere spezifische Aktivität aufweist.
(iii) Das native Gen für Anthranilat-Phosphoribosyltransferase-Aktivität kann durch ein anderes Gen, das von einem verschiedenen Organismus stammt, ersetzt und ein Enzym mit einer spezifischen Anthranilat-Phosphoribosyltransferase-Aktivität, die geringer ist als die der zuvor erwähnten nativen Gene (z.B. TRP4, *trpD* oder *trpDC),* kodiert werden.

Unabhängig davon, welcher Mikroorganismus eingesetzt wird, umfasst die Fermentationsbrühe zu Beginn der Fermentation in Schritt (I-1) rekombinante Zellen des eingesetzten Mikroorganismus und mindestens eine fermentierbare Kohlenstoff-haltige Verbindung (sowie mindestens eine Stickstoff-haltige Verbindung als Stickstoffquelle). Bevorzugt enthält die Fermentationsbrühe darüber hinaus noch weitere Bestandteile ausgewählt aus der Gruppe bestehend aus Puffersystemen, anorganischen Nährstoffen, Aminosäuren, Vitaminen und weiteren organischen Verbindungen welche für das Wachstum bzw. den Erhaltungsstoffwechsel der rekombinanten Zellen benötigt werden. Die Fermentationsbrühe ist wasserbasiert. Nach Einsetzen des Fermentationsprozesses umfasst die Fermentationsbrühe auch Aminobenzoesäure, das angestrebte Fermentationsprodukt.

Unter einer fermentierbaren Kohlenstoff-haltigen Verbindung im Sinne der vorliegenden Erfindung wird jede organische Verbindung oder Mischung organischer Verbindungen verstanden, die von den rekombinanten Zellen des eingesetzten Mikroorganismus verwendet werden kann, um Aminobenzoesäure zu produzieren. Die Produktion von Aminobenzoesäure kann dabei in Gegenwart oder Abwesenheit von Sauerstoff stattfinden.

Bevorzugt sind dabei solche fermentierbaren Kohlenstoff-haltigen Verbindungen, die zusätzlich als Energie- und Kohlenstoffquelle für das Wachstum der rekombinanten Zellen des eingesetzten Mikroorganismus dienen können. Besonders geeignet sind Stärkehydrolysat, Zuckerrohrsaft, Zuckerrübensaft und Hydrolysate aus lignocellulosehaltigen Rohstoffen. Ebenfalls geeignet sind Glycerin und C1-Verbindungen, insbesondere Kohlenstoffmonoxid.

In einer bevorzugten Ausführungsform wird Schritt (I-1) kontinuierlich durchgeführt, d. h. dass die Edukte dem Fermentationsreaktor kontinuierlich zugeführt und das Produkt dem Fermentationsreaktor kontinuierlich entnommen wird. In kontinuierlicher Verfahrensführung wird der Mikroorganismus unter Umständen mit dem Produktstrom ausgetragen; der Mikroorganismus reproduziert sich jedoch im Allgemeinen selbst, sodass eine Zuführung von frischem Mikroorganismus in der Regel nicht nötig ist (erforderlichenfalls aber natürlich vorgenommen werden kann). Eine Zellrückhaltung zur Vermeidung der Austragung von Mikroorganismus ist ebenfalls möglich.

In einer anderen bevorzugten Ausführungsform wird Schritt (I-1) in einer diskontinuierlichen Verfahrensführung (sog. "Batch-Fahrweise") durchgeführt. In einer Variante der diskontinuierlichen Fahrweise (sog. "*Fed*-Batch-Fahrweise") werden die Edukte dem Fermentationsreaktor so lange kontinuierlich zugeführt, wie es das Reaktorvolumen erlaubt, ohne dass Produkte dem Reaktor entnommen werden. Die Reaktion wird nach Zugabe der maximal möglichen Menge an Edukten unterbrochen und das Produktgemisch dem Fermentationsreaktor entnommen.

Unabhängig von der genauen Fahrweise umfasst der Fermentationsreaktor bevorzugt Einrichtungen zur Messungen wichtiger Prozessparameter wie Temperatur, pH-Wert der Fermentationsbrühe, Konzentration an Substrat und Produkt, Gehalt an gelöstem Sauerstoff, Zelldichte der Fermentationsbrühe. Insbesondere bevorzugt umfasst der Fermentationsreaktor Einrichtungen zur Anpassung von mindestens einem (bevorzugt von allen) der vorgenannten Prozessparameter.

Geeignete Fermentationsreaktoren sind Rührkessel, Membranreaktoren, Kolbenstromreaktoren ("plug flow reactors") oder Schlaufenreaktoren (siehe beispielsweise Bioprozesstechnik, Horst Chmiel, ISBN-10: 3827424763, Spektrum Akademischer Verlag). Besonders bevorzugt sowohl für aerobe als auch für anaerobe Fermentationen sind Rührkesselreaktoren und Schlaufenreaktoren (insbesondere Airliftreaktoren, in denen die Zirkulation der Flüssigkeit im Reaktor durch Begasung erreicht wird).

Bevorzugt umfasst der Schritt (I) neben dem Schritt (I-1) auch eine *Aufarbeitung der erhaltenen Fermentationsbrühe* in einem Schritt (1-2). Diese Aufarbeitung umfasst bevorzugt die folgenden Schritte:
(α) Abtrennung des Mikroorganismus aus der in Schritt (I-1) erhaltenen Fermentationsbrühe und/oder
(β) Entfärbung der in Schritt (I-1) erhaltenen Fermentationsbrühe oder, bei Durchführung von Schritt (α), der in Schritt (α) erhaltenen an Mikroorganismen abgereicherten Fermentationsbrühe.

Die *Abtrennung des Mikroorganismus aus der Fermentationsbrühe* in Schritt (α) ist an sich aus dem Stand der Technik bekannt und erfolgt im Rahmen der vorliegenden Erfindung insbesondere durch Filtration, Absetzen (sog. Settling), Abtrennung in Hydrozyklonen oder Zentrifugation. Eine mögliche Ausgestaltung dieses Schrittes ist in WO 2015/124686 A1 und WO 2015/124687 A1 beschrieben. Dabei wird insbesondere auf WO 2015/124687 A1, Seite 15, Zeile 8 bis Seite 15, Zeile 17, verwiesen.

Unabhängig davon, ob der Mikroorganismus abgetrennt wird oder nicht, kann Schritt (I-2) erforderlichenfalls einen Schritt (β) zur *Entfärbung der Fermentationsbrühe bzw. der an Mikroorganismen abgereicherten Fermentationsbrühe* umfassen. Dieser Schritt (β) wird in einer Ausführungsform bevorzugt so durchgeführt, dass Fermentationsbrühe bzw. an Mikroorganismen abgereicherte Fermentationsbrühe über eine Säule mit fester Packung geleitet wird, um Farbstoffe mittels Adsorption zu entfernen. Als mögliche feste Phase kann z. B. Kieselgur, Aktivkohle oder eine Ionenaustauscherpackung verwendet werden. Diese Ausführungsform ist insbesondere dann bevorzugt, wenn Schritt (α) durchgeführt wird. In einer anderen Ausführungsform wird die in Schritt (I-1) erhaltene Fermentationsbrühe unter Zusatz von pulverförmiger Aktivkohle oder pulverförmigen Kieselgur gerührt und dann filtriert.

Schritt (β) wird bevorzugt dann durchgeführt, wenn in der Fermentationsbrühe bzw. der an Mikroorganismen abgereicherten Fermentationsbrühe aus Schritt (α) solche farbigen Substanzen vorhanden sind, welche die nachfolgenden Schritte des erfindungsgemäßen Verfahrens, insbesondere die in bevorzugten Ausführungsformen durchgeführte und im Detail noch zu beschreibende *Kristallisation,* stören könnten.

Zunächst werden *bevorzugte Ausführungsformen* der Schritte (II) und (III) geschildert.

In **Schritt (II)** erfolgt die Adsorption der in Schritt (I) bereitgestellten wässrigen Lösung der Aminobenzoesäure **(Adsorptionsschritt).**

Diese Adsorption erfolgt in allen Varianten bevorzugt bei einem pH-Wert im Bereich von > 3,0 bis 4,0, besonders bevorzugt im Bereich von 3,3 bis 3,7 und ganz besonders bevorzugt bei pH 3,5. Die Temperatur liegt bevorzugt im Bereich von 15 °C bis 40 °C, besonders bevorzugt im Bereich von 15 °C bis 35 °C und ganz besonders bevorzugt im Bereich von 20 C bis 25 °C. Die Adsorption erfolgt bevorzugt an Aktivkohle als Adsorptionsmittel. Als Apparate für die Adsorption eignen sich insbesondere Kolonnen, im Labormaßstab zweckmäßigerweise Chromatographiesäulen, mit denen das Adsorptionsmittel beschickt wird, insbesondere in Form eine Adsorberbetts.

In **Schritt (III)** erfolgt die Desorption der in Schritt (II) adsorbierten Aminobenzoesäure **(Desorptionsschritt)**.

Die Desorption erfolgt in allen Varianten bei einem pH-Wert im Bereich von -0,8 bis 3,0, bevorzugt -0,5 bis 3,0, besonders bevorzugt 0,1 bis 3,0, ganz besonders bevorzugt im Bereich von 0,5 bis 2,5 und außerordentlich ganz besonders bevorzugt im Bereich von 1,0 bis 2,0 (d. h. das eingesetzte Desorptionsmittel weist - bei 20 °C - einen pH-Wert in den genannten Bereichen auf). Damit ist gemeint, dass die Desorption der Hauptmenge der Aminobenzoesäure (d. h. mindestens 90 %, bevorzugt 95 %, insbesondere > 99 %, der Gesamtmenge an in Schritt II) adsorbierter Aminobenzoesäure) in diesen pH-Bereichen erfolgt. Eine Desorption der Hauptmenge der Aminobenzoesäure bei anderen pH-Werten, gefolgt von einer sauren Wäsche des Adsorptionsmittels in den genannten pH-Bereichen ist hiervon nicht umfasst. Als wässriges Desorptionsmittel eignet sich insbesondere eine saure wässrige Lösung, insbesondere Salzsäure [HCl(aq)]. Bevorzugt verbleibt das beladene Adsorptionsmittel im Desorptionsschritt in dem für die Adsorption eingesetzten Apparat (insbesondere eine Kolonne). In der großtechnischen Produktion werden zu diesem Zweck bevorzugt mindestens zwei mit Adsorptionsmittel beschickte Kolonnen vorgehalten, in denen die Schritte (III) und (IV) im Wechsel durchgeführt werden.

In einer ersten Ausführungsform wird das wässrige Desorptionsmittel in entgegengesetzter Richtung durch die in der Adsorption eingesetzten Kolonne geführt wie zuvor die wässrige Lösung an Aminobenzoesäure. Wenn die im Desorbat bestimmte Konzentration an Aminobenzoesäure ein Minimum erreicht (d. h. nicht mehr weiter sinkt), wird die Zufuhr des wässrigen Desorptionsmittels gestoppt. Die Konzentrationsbestimmung erfolgt im Labormaßstab durch regelmäßige Probenahme und Analyse, bevorzugt mittels Flüssigchromatographie mit UV-Detektor. Im großtechnischen Prozess können UV-Onlineanalysatoren eingesetzt werden.

Im Anschluss wird im Adsorptionsmittel (im Adsorberbett) noch vorhandenes Desorbat eluiert, bevorzugt mit Wasser (insbesondere mit vollentsalztem Wasser), Fermentationsbrühe oder Mutterlauge aus der (weiter unten noch im Detail beschriebenen) Kristallisation, bevorzugt Fermentationsbrühe oder Mutterlauge aus der Kristallisation. Dies geschieht bevorzugt bei einer Temperatur im Bereich von 15 °C bis 40 °C, besonders bevorzugt im Bereich von 15 °C bis 35 °C und ganz besonders bevorzugt im Bereich von 20 °C bis 25 °C. Danach wird das Adsorberbett bevorzugt gespült, und zwar bevorzugt mit Wasser (insbesondere mit vollentsalztem Wasser), Fermentationsbrühe oder Mutterlauge aus der Kristallisation, bevorzugt Fermentationsbrühe oder Mutterlauge aus der Kristallisation, ebenfalls bei einer Temperatur im Bereich von 15 °C bis 40 °C, besonders bevorzugt im Bereich von 15 °C bis 35 °C und ganz besonders bevorzugt im Bereich von 20 °C bis 25 °C. Die erhaltene Spülflüssigkeit kann verworfen oder - bevorzugt - in den Prozess zurückgeführt werden, und zwar dergestalt, dass die Spülflüssigkeit zusammen mit der wässrigen Lösung an Aminobenzoesäure dem Adsorptionsschritt (II) zugeführt wird. Alternativ zur beschriebenen Spülung ist es auch möglich, durch das Adsorberbett Wasser (insbesondere mit vollentsalztes Wasser), Fermentationsbrühe oder Mutterlauge aus der Kristallisation, bevorzugt Fermentationsbrühe oder Mutterlauge aus der Kristallisation, im Kreis zu führen und dabei so lange eine wässrige Basenlösung (bevorzugt ausgewählt aus der Gruppe bestehend aus Ammoniakwasser, Natronlauge, Kalilauge und Calciumhydroxidlösung, insbesondere Natronlauge) zuzudosieren, bis der für den Adsorptionsschritt (II) angestrebte pH-Wert (der bevorzugt im Bereich von > 3,0 bis 4,0, besonders bevorzugt im Bereich von 3,3 bis 3,7 und ganz besonders bevorzugt bei pH 3,5 liegt) erreicht ist.

In einer anderen Ausführungsform wird das Desorbat bis zum Erreichen des Gleichgewichts durch die mit Adsorptionsmittel beschickte Kolonne im Kreis geführt. Das Gleichgewicht ist erreicht, wenn die im Desorbat gemessene Konzentration an Aminobenzoesäure ein Maximum erreicht, d. h. den Wert annimmt, der bei der gewählten Desorptionsmittelmenge und dem vorliegenden Beladungsgrad des Adsorptionsmittels maximal erreicht werden kann. Die Konzentrationsbestimmung erfolgt im Labormaßstab durch regelmäßige Probenahme und Analyse, bevorzugt mittels Flüssigchromatographie mit UV-Detektor. Im großtechnischen Prozess können UV-Onlineanalysatoren eingesetzt werden. Die Elution des im Adsorptionsmittel vorhandenen Desorbats erfolgt bevorzugt wie zuvor für die erste Ausführungsform beschrieben.

Die Temperatur im Desorptionschritt liegt in allen Ausführungsformen bevorzugt im Bereich von 50 °C bis 90 °C, besonders bevorzugt im Bereich von 60 °C bis 85 °C und ganz besonders bevorzugt im Bereich von 70 °C bis 80 °C. Aufgrund des niedrigen pH-Werts wird die Aminobenzoesäure in der kationischen (protonierten) Form desorbiert.

Erfindungsgemäß wird die Kristallisation *im Anschluss* an Schritt (I-1) bzw. - sofern durchgeführt - an Schritt (I-2) durchgeführt. Die Erfindung umfasst demnach ein Verfahren, bei welchem Schritt (I) weiterhin umfasst:
(I-3) Einführen der in Schritt (I-1) oder, sofern durchgeführt, in Schritt (I-2), erhaltenen Fermentationsbrühe in einen Reaktor, in dem durch Vermischen mit einer sauren wässrigen Lösung Aminobenzoesäure abgeschieden wird, wobei bevorzugt der pH-Wert der resultierenden Mischung auf einen Wert im Bereich von 3,0 bis 4,7, bevorzugt im Bereich von 3,2 bis 3,7, besonders bevorzugt im Bereich von 3,4 bis 3,6, eingestellt wird;
(I-4) Abtrennung der in Schritt (I-3) abgeschiedenen Aminobenzoesäure, bevorzugt durch Filtration, wobei eine an Aminobenzoesäure abgereicherte Mutterlauge erhalten wird;
(I-5) optionale weitere Reinigung der in Schritt (I-4) abgetrennten Aminobenzoesäure, bevorzugt durch Waschen mit Wasser.

Die in Schritt (II) eingesetzte wässrige Lösung von Aminobenzoesäure ist also die in Schritt (I-4) erhaltene an Aminobenzoesäure abgereicherte **Mutterlauge.** Die Sequenz aus Adsorption (Schritt (II)) und Desorption (Schritt (III)) dient also dazu, die in der Kristallisation (*Schritt (I-3)*) erhaltene Mutterlauge weiter an darin gelöster Aminobenzoesäure abzureichern und somit die Ausbeute zu erhöhen.

Da die Kristallisation durch Zugabe *von Säure* eingeleitet wird (Schritt (1-3)), ist es vorgesehen, das in Schritt (III) erhaltene, an Aminobenzoesäure angereicherte Desorbat (das stark sauer ist - pH-Wert im Bereich von -0,8 bis 3,0, bevorzugt -0,5 bis 3,0, besonders bevorzugt 0,1 bis 3,0, ganz besonders bevorzugt 0,5 bis 2,5, außerordentlich ganz besonders bevorzugt 1,0 bis 2,0) als Bestandteil, ggf. alleinigen Bestandteil, der in Schritt (I-3) eingesetzten sauren wässrigen Lösung zu verwenden. In der vorliegenden Erfindung wird die Aminobenzoesäure demnach durch Kristallisation aus dem Desorbat gewonnen, wobei *die aus dem Desorbat zu gewinnende Aminobenzoesäure Teil des Fällungsmittels (der zugesetzten sauren wässrigen Lösung) ist.* **Schritt** (**IV**) - die **Gewinnung der Aminobenzoesäure aus dem Desorbat** - entspricht daher *dem Schritt (I-3).*

Es ist auch möglich, zur Fällung in Schritt (I-3) zusätzlich eine Säure aus einer externen Quelle zu verwenden, bevorzugt Salzsäure, Schwefelsäure und/oder Phosphorsäure, insbesondere Salzsäure einer Konzentration von 15 Massen-% bis 37 Massen-%. Diese Säure aus einer externen Quelle ist dann neben dem bereits erwähnten Desorbat ein Bestandteil der sauren wässrigen Lösung. Weitere Säuren werden bevorzugt nicht eingesetzt.

Für den Schritt der **Kristallisation** gibt es *bevorzugte Ausführungsformen,* die im Folgenden näher geschildert werden.

In einem ersten Schritt (entsprechend Schritt (1-3)), der Kristallisation selbst, wird durch Säurezugabe zur jeweiligen Lösung von Aminobenzoesäure der pH-Wert so eingestellt, dass Aminobenzoesäure (d. h. Aminobenzoesäure in der neutralen Form) auskristallisiert. Diese Art der Kristallisation wird auch als *Reaktiv*kristallisation bezeichnet. Dies geschieht bevorzugt derart, dass der pH-Wert der resultierenden Mischung dem des isoelektrischen Punkts des abzuscheidenden Isomers von Aminobenzoesäure entspricht oder diesem zumindest nahekommt. Im Falle von ortho-Aminobenzoesäure als gewünschtem Produkt wird daher der pH-Wert bevorzugt auf einen Wert im Bereich von 3,0 bis 4,7, besonders bevorzugt auf einen Wert im Bereich von 3,2 bis 3,7, ganz besonders bevorzugt auf einen Wert im Bereich von 3,4 bis 3,6, eingestellt, also nahe oder entsprechend dem isoelektrischen Punkt bei pH 3,5. Dieser isoelektrische Punkt liegt für die beiden anderen Isomere von Aminobenzoesäure jeweils bei etwa pH 3,7. Die pH-Wert-Einstellung in diesem Schritt ist dabei bevorzugt "*einstufig"* in dem Sinne, dass der gewünschte Ziel-pH-Wert durch Säure- oder Basenzugabe direkt eingestellt wird, ohne dass bei pH-Werten zwischen dem Ausgangs-pH-Wert und dem Ziel-pH-Wert Zwischenschritte (wie Filtration, Zentrifugation, säulenchromatographische Behandlung und dgl.) durchgeführt werden.

Als Reaktor für diesen Schritt kommen dem Fachmann geläufige übliche Ausgestaltungen chemischer Reaktoren in Betracht. Beispielhaft seien Rührkessel oder Zwangsumlaufkristallisatoren wie solche vom "Typ Oslo" genannt. Die Zugabe der Säure oder Base in den Reaktor erfolgt dabei bevorzugt kontinuierlich. Das Verfahrensprodukt dieses Schrittes - also in saurer Mutterlauge suspendierte Aminobenzoesäure - wird dabei dem Reaktor zumindest absatzweise oder bevorzugt ebenfalls kontinuierlich entnommen. In diesem Fall erfolgt vorzugsweise auch die weitere Behandlung sich anschließenden Schritt absatzweise oder kontinuierlich.

In einem zweiten Schritt (entsprechend Schritt (I-4)) wird die im vorigen Schritt abgeschiedene Aminobenzoesäure abgetrennt. Methoden hierzu sind an sich aus dem Stand der Technik bekannt. Erfindungsgemäß erfolgt dieser Schritt bevorzugt durch Filtration oder Zentrifugation. Bevorzugt wird dieser Schritt durchgeführt wie in WO 2015/124687 A1 beschrieben. Dabei wird insbesondere auf WO 2015/124687 A1, Seite 17, Zeile 13 bis Seite 17, Zeile 16, verwiesen. Eine Filtration kann bei vermindertem Druck, Umgebungsdruck oder erhöhtem Druck durchgeführt werden. Eine Zentrifugation kann mit handelsüblichen Zentrifugen durchgeführt werden. Es ist auch möglich, die im ersten Schritt erhaltene Suspension ruhen zu lassen, bis sich die ausgefallenen Kristalle an Aminobenzoesäure absetzen, und dann die überstehende Mutterlauge abzudekantieren oder abzusaugen.

In einem optionalen dritten Schritt (entsprechend Schritt (I-5)) schließt sich eine weitere Reinigung der zuvor gewonnenen Aminobenzoesäure an. Auch dieser Schritt ist an sich aus dem Stand der Technik bekannt (siehe vor allem WO 2015/124687 A1 und insbesondere auf WO 2015/124687 A1, Seite 18, Zeile 4 bis Seite 18, Zeile 6) und erfolgt bevorzugt durch eine oder mehrere Wäschen mit wässrigen Waschmedien, insbesondere Wasser. Um Ausbeuteverluste zu vermeiden, kann der pH-Wert des wässrigen Waschmediums auf den gleichen Wert im ersten Schritt nach beendeter Säure- bzw. Basenzugabe eingestellt werden; es wird also in dieser Ausführungsform statt mit Wasser mit einer verdünnten Säure oder verdünnten Base, insbesondere der gleichen Säure bzw. Base wie im Fällungsreagenz eingesetzt, gewaschen.

In Schritt (IV) wird die Aminobenzoesäure in der neutralen Form (also als Aminobenzoesäure "im engeren Sinne") gewonnen. In dieser Form kann sie unmittelbar der optionalen (und bevorzugten) Umsetzung zu einem Aminobenzoesäurefolgeprodukt in **Schritt** (**V**) zugeführt werden. Ausgewählte weitere Umsetzungen des erhaltenen Anilins in Schritt (IV) sind:
(V-1) Decarboxylierung der Aminobenzoesäure zu Anilin;
(V-2) Decarboxylierung der Aminobenzoesäure zu Anilin, gefolgt von säurekatalysierter Reaktion des Anilins mit Formaldehyd unter Bildung von Di- und Polyaminen der Diphenylmethanreihe;
(V-3) Decarboxylierung der Aminobenzoesäure zu Anilin, gefolgt von säurekatalysierter Reaktion des Anilins mit Formaldehyd unter Bildung von Diund Polyaminen der Diphenylmethanreihe, gefolgt von Umsetzung mit Phosgen unter Bildung von Di- und Polyisocyanaten der Diphenylmethanreihe;
(V-4) Decarboxylierung der Aminobenzoesäure zu Anilin, gefolgt von Umsetzung des Anilins zu einer Azoverbindung;
(V-5) Umsetzung der Aminobenzoesäure zu einem Amid;
(V-6) Umsetzung der Aminobenzoesäure zu einem leitenden Polymeren wie insbesondere Polyanthranilsäure.

Die Decarboxylierung der Aminobenzoesäure zu **Anilin (V-1)** ist an sich bekannt und kann nach einem beliebigen Verfahren des Standes der Technik durchgeführt werden. Bevorzugt ist die folgende Vorgehensweise:
Schritt (V-I) kann in allen in der Verfahrenstechnik üblichen, dem Fachmann geläufigen Reaktortypen wie bspw.
- Rührkesselreaktoren (bevorzugt mit Katalysatorfestbett),
- kontinuierlich betriebene Rührkesselreaktoren (in der englischen Literatur als *continuous stirred tank reactors* (CSTR) bezeichnet), insbesondere kontinuierlich betriebene Rührkesselreaktoren (CSTR) mit Katalysatorfestbett,
- Kolbenstromreaktoren (in der englischen Literatur als *plug flow reactors* bezeichnet) mit Katalysatorfestbett oder
- Schlammphasenreaktoren (auch Suspensionsreaktoren genannt; in der englischen Literatur als *slurry phase reactors* bezeichnet) mit Katalysator-Rezirkulation bzw. Katalysator-Rückgewinnung
durchgeführt werden.

Es können auch mehrere Reaktoren zu einer Reaktorkaskade hintereinandergeschaltet werden, d. h. der flüssige Produktaustrag eines Reaktors fließt zur weiteren Umsatzvervollständigung in den nächstfolgenden Reaktor.

Schritt (V-I) wird bevorzugt in Gegenwart eines Katalysators durchgeführt. Als Katalysatoren für die Durchführung von Schritt (V-I) eignen sich dem Fachmann geläufige Katalysatoren wie bspw. wässrige Säuren wie Schwefelsäure, Salpetersäure und Salzsäure; feste Säuren wie Zeolithe und Si-Ti-Molekularsiebe, feste Basen wie Hydroxy-Apatite und Hydrotalcite; polymere Säuren wie Ionenaustauscherharze (insbesondere Amberlyst). Wird der Katalysator in Form von Partikeln oder in Pulverform eingesetzt, so besteht eine bevorzugte Ausführungsform der Erfindung darin, den Katalysator in der flüssigen Reaktionsmischung aufzuschlämmen, bevorzugt durch Rühren. Hierzu eignet sich besonders ein Schlammphasenreaktor (auch Suspensionsreaktor genannt), wobei der Katalysator in einer Konzentration im Bereich von 0,100 Massen-% bis 50,0 Massen-%, bevorzugt im Bereich von 10,0 Massen-% bis 30,0 Massen-%, bezogen auf die Gesamtmasse der flüssigen Reaktionsmischung, eingesetzt wird. In einer anderen bevorzugten Ausführungsform wird der Katalysator in einem Katalysatorbett in einem Rohrreaktor angeordnet, wobei der in dieser Ausführungsform insbesondere in Partikeln (bspw. Kugeln) vorliegende Katalysator vorzugsweise im Katalysatorbett fixiert ist, bspw. zwischen Siebplatten angeordnet ist. Unabhängig von der Art des eingesetzten Reaktors wird als in Schritt (I) eingesetzter Katalysator vorzugsweise ein ZeolithKatalysator verwendet, besonders bevorzugt ein Zeolith vom Typ Y in der protonierten Form (H-Form). Die Anordnung des, insbesondere in Partikelform vorliegenden, Katalysators in einem fixierten Bett ist natürlich nicht auf Rohrreaktoren beschränkt, sondern kann prinzipiell auch bei gerührten Reaktoren zur Anwendung kommen. Ferner ist es möglich, den Katalysator in monolithischer Form einzusetzen.

In der Decarboxylierung von Schritt (V-I) können bspw. die folgenden Reaktionsparameter eingehalten werden:
- Temperatur bevorzugt im Bereich von 140 °C bis 240 °C und Druck bevorzugt im Bereich von 1,00 bar_{(abs.)} bis 20,0 bar_{(abs.)},
- Temperatur besonders bevorzugt im Bereich von 160 °C bis 220 °C und Druck besonders bevorzugt im Bereich von 1,00 bar_{(abs.)} bis 15,0 bar_{(abs.)},
- Temperatur ganz besonders bevorzugt bei einer Temperatur im Bereich von 180 °C bis 200 °C und Druck ganz besonders bevorzugt im Bereich von 4,00 bar_{(abs.)} bis 10,0 bar_{(abs.)}.

Bevorzugt durchläuft der Anilin enthaltende Strom vor seiner Entnahme aus dem Reaktor ein Filter, um zu verhindern, dass Feststoffpartikel (bspw. Katalysatorpartikel) mitgerissen werden.

Die zu decarboxylierende Aminobenzoesäure wird für die Durchführung des Schrittes (V-1) bevorzugt in Lösung eingesetzt. Als Lösungsmittel eignen sich Wasser oder organische Lösungsmittel wie 1-Dodecanol oder Anilin.

Schritt (V-I) wird bevorzugt kontinuierlich durchgeführt, d. h. dass die zu decarboxylierende Aminobenzoesäure dem Reaktor kontinuierlich zugeführt und das Produkt dem Reaktor kontinuierlich entnommen wird. In einer Variante dieser Verfahrensweise werden auch mindestens Teile des Katalysators im kontinuierlichen Betrieb permanent oder intervallweise gewechselt, um eine Erschöpfung seiner Leistungsfähigkeit zu verhindern. Eine diskontinuierliche Verfahrensführung (sog. "Batch-Fahrweise") ist aber auch möglich. In einer Variante der diskontinuierlichen Fahrweise (sog. "*Fed*-Batch-Fahrweise") werden die Edukte dem Reaktor so lange kontinuierlich zugeführt, wie es das Reaktorvolumen erlaubt, ohne dass Produkte dem Reaktor entnommen werden. Die Reaktion wird nach Zugabe der maximal möglichen Menge an Edukten unterbrochen und das Produktgemisch dem Reaktor entnommen.

In einer alternativen bevorzugten Ausführungsform ist auch eine Verfahrensführung denkbar, bei welcher zu decarboxylierende Aminobenzoesäure dem Reaktor kontinuierlich zugeführt und das Produkt dem Reaktor kontinuierlich entnommen wird, verbrauchter Katalysator jedoch nicht im kontinuierlichen Betrieb entnommen wird, sondern stattdessen so lange frischer Katalysator (entweder permanent oder intervallweise) zugesetzt wird, bis die durch das vorhandene Reaktorvolumen vorgegebene maximale Katalysatormenge im Reaktor erreicht ist, und dann der Reaktor zwecks Reinigung und Katalysatorwechsel außer Betrieb genommen wird.

In allen Ausführungsformen ist es bevorzugt, Schritt (V-I) unter Sauerstoffausschluss durchzuführen. Zur Inertisierung des Reaktors eignen sich inerte Gase wie Stickstoff, Kohlenstoffdioxid oder Edelgase.

Das dem Reaktor von Schritt (V-1) entnommene Rohanilin wird vorzugsweise vor seiner weiteren Verwendung aufgereinigt. Diese Aufreinigung kann durch dem Fachmann geläufige Verfahren erfolgen. Insbesondere umfasst die Aufreinigung mindestens einen Destillationsschritt, welchem eine Wasserabtrennung durch Phasentrennung vorgeschaltet sein kann. Die Aufreinigung kann ebenfalls eine Basenbehandlung zur Entfernung saurer Verunreinigungen vor, während oder nach dem Destillationsschritt umfassen. Geeignete Ausgestaltungen sind bspw. in EP-A-1 845 079, EP-A-1 845 080, EP-A-2 263 997 und EP-A-2 028 176 beschrieben. (Diese Schriften befassen sich mit der Reinigung von Anilin, das durch Hydrierung von Nitrobenzol erhalten wurde; die beschriebenen Reinigungsschritte des Rohanilins sind jedoch auf anders hergestelltes Anilin ebenfalls anwendbar.)

Die weitere Umsetzung so gewonnenen Anilins mit Formaldehyd zu **Di- und Polyaminen der Diphenylmethanreihe** (**V-2**) ist an sich bekannt und kann nach einem beliebigen Verfahren des Standes der Technik durchgeführt werden. Die kontinuierliche oder teilweise diskontinuierliche Herstellung von Di- und Polyaminen der Diphenylmethanreihe aus Anilin und Formaldehyd ist z. B. in EP 1 616 890 A1, US-A-5286760, EP-A-451442 und WO-A-99/40059 offenbart. Die Reaktion erfolgt unter Säurekatalyse. Als saurer Katalysator eignet sich vorzugsweise Salzsäure.

Die weitere Umsetzung der so erhaltenen Di- und Polyamine der Diphenylmethanreihe mit Phosgen zu **Di- und Polyisocyanaten der Diphenylmethanreihe** (**V-3**) ist ebenfalls an sich bekannt und kann nach einem beliebigen Verfahren des Standes der Technik durchgeführt werden. Geeignete Verfahren sind beispielsweise in EP 2 077 150 B1, EP 1 616 857 A1, EP 1 873 142 A1, und EP 0 314 985 B1 beschrieben.

Die Umsetzung des durch Decarboxylierung der erfindungsgemäß erhaltenen Aminobenzoesäure gewonnen Anilins zu **Azoverbindungen, insbesondere zu Azo-Farbstoffen** (**V-4**) kann nach einem beliebigen Verfahren des Standes der Technik erfolgen. Beispielhaft sei auf die bekannte Herstellung von Anilingelb (para-Aminoazobenzol; CAS 493-5-7) oder Indigo (2,2'-Bis(2,3-dihydro-3-oxomethyliden); CAS 482-89-3) verwiesen (Per Wiklund et al., Current Organic Synthesis, 2006, 3, 379 - 402).

Die Umsetzung der erfindungsgemäß erhaltenen Aminobenzoesäure zu einem **Amid** (**V-5**) kann nach einem beliebigen Verfahren des Standes der Technik erfolgen. Beispielhaft sei das primäre Amin von Anthranilsäure (2-Aminobenzylamid) erwähnt, welches unter anderem als Startmaterial für die Herstellung von Pharmazeutika verwandt wird (Per Wiklund et al., Current Organic Synthesis, 2006, 3, 379 - 402).

Die Umsetzung der erfindungsgemäß erhaltenen Aminobenzoesäure zu einem **leitenden Polymeren wie insbesondere Polyanthanilsäure (V-6)** kann nach einem beliebigen Verfahren des Standes der Technik erfolgen. Ein Beispiel ist in Bhavana Guptaa et al., Polymers Advanced Technologies, 2011, 22, 1982-1988, beschrieben.

## Patentansprüche

1. Verfahren zur Herstellung von Aminobenzoesäure oder eines Aminobenzoesäurefolgeprodukts, umfassend die Schritte:
(I) Bereitstellen einer wässrigen Lösung von Aminobenzoesäure unter Anwendung eines fermentativen Verfahrens, wobei Schritt (I) umfasst:
(I-1) Fermentation eines Rohstoffes, der wenigstens
• eine fermentierbare Kohlenstoff-haltige Verbindung, bevorzugt ausgewählt aus der Gruppe bestehend aus Stärkehydrolysat, Zuckerrohrsaft, Zuckerrübensaft und Hydrolysaten aus lignocellulosehaltigen Rohstoffen, und
• eine Stickstoff-haltige Verbindung, bevorzugt ausgewählt aus der Gruppe bestehend aus Ammonikgas, Ammoniakwasser, Ammoniumsalzen (insbesondere Ammoniumsulfat und Ammoniumchlorid) und Harnstoff, umfasst,
in einem Fermentationsreaktor unter Verwendung von Mikroorganismen unter Erhalt einer Fermentationsbrühe; woran sich optional
(I-2) eine Aufarbeitung umfassend die folgenden Aufarbeitungsschritte:
(α) Abtrennung des Mikroorganismus aus der in Schritt (I-1) erhaltenen Fermentationsbrühe
und/oder
(β) Entfärbung der in Schritt (I-1) erhaltenen Fermentationsbrühe oder, bei Durchführung von Schritt (α), der in Schritt (α) erhaltenen, an Mikroorganismen abgereicherten, Fermentationsbrühe anschließt,
(I-3) Einführen der in Schritt (I-1) oder, sofern durchgeführt, in Schritt (I-2), erhaltenen Fermentationsbrühe in einen Reaktor, in dem durch Vermischen mit einer sauren wässrigen Lösung Aminobenzoesäure abgeschieden wird, wobei bevorzugt der pH-Wert der resultierenden Mischung auf einen Wert im Bereich von 3,0 bis 4,7, bevorzugt im Bereich von 3,2 bis 3,7, besonders bevorzugt im Bereich von 3,4 bis 3,6, eingestellt wird;
(I-4) Abtrennung der in Schritt (I-3) abgeschiedenen Aminobenzoesäure, bevorzugt durch Filtration, wobei eine an Aminobenzoesäure abgereicherte Mutterlauge erhalten wird;
(II) Behandlung der in Schritt (I) bereitgestellten wässrigen Lösung von Aminobenzoesäure, nämlich der in Schritt (I-4) erhaltenen an Aminobenzoesäure abgereicherten Mutterlauge, mit einem Adsorptionsmittel, vorzugsweise Aktivkohle, wobei das Adsorptionsmittel mit Aminobenzoesäure beladen wird und ein an Aminobenzoesäure abgereichertes Adsorbat erhalten wird;
(III) Behandlung des mit Aminobenzoesäure beladenen Adsorptionsmittels aus Schritt (II) mit einem wässrigen Desorptionsmittel eines pH-Werts im Bereich von -0,8 bis 3,0, bevorzugt -0,5 bis 3,0, besonders bevorzugt 0,1 bis 3,0, ganz besonders bevorzugt 0,5 bis 2,5, außerordentlich ganz besonders bevorzugt 1,0 bis 2,0, wobei ein an Aminobenzoesäure angereichertes Desorbat und ein an Aminobenzoesäure abgereichertes Adsorptionsmittel erhalten werden;
(IV) Gewinnung der Aminobenzoesäure aus dem in Schritt (III) erhaltenen Desorbat durch Verwendung des in Schritt (III) erhaltenen an Aminobenzoesäure angereicherten Desorbats als Bestandteil der in Schritt (I-3) eingesetzten sauren wässrigen Lösung;
(V) optionale weitere Umsetzung der in Schritt (IV) gewonnenen Aminobenzoesäure zu einem Aminobenzosäurefolgeprodukt, wobei Schritt (V) insbesondere eine der folgenden Umsetzungen umfasst:
(V-1) Decarboxylierung der Aminobenzoesäure zu Anilin;
(V-2) Decarboxylierung der Aminobenzoesäure zu Anilin, gefolgt von säurekatalysierter Reaktion des Anilins mit Formaldehyd unter Bildung von Di- und Polyaminen der Diphenylmethanreihe;
(V-3) Decarboxylierung der Aminobenzoesäure zu Anilin, gefolgt von säurekatalysierter Reaktion des Anilins mit Formaldehyd unter Bildung von Di- und Polyaminen der Diphenylmethanreihe, gefolgt von Umsetzung mit Phosgen unter Bildung von Di- und Polyisocyanaten der Diphenylmethanreihe;
(V-4) Decarboxylierung der Aminobenzoesäure zu Anilin, gefolgt von Umsetzung des Anilins zu einer Azoverbindung;
(V-5) Umsetzung der Aminobenzoesäure zu einem Amid;
(V-6) Umsetzung der Aminobenzoesäure zu leitenden Polymeren wie insbesondere Polyanthranilsäure.

2. Verfahren gemäß Anspruch 1, bei welchem Schritt (I) weiterhin umfasst:
(I-5) eine weitere Reinigung der in Schritt (I-4) abgetrennten Aminobenzoesäure, bevorzugt durch Waschen mit Wasser.

3. Verfahren gemäß Anspruch 1 oder 2, bei welchem das in Schritt (III) erhaltene, an Aminobenzoesäure angereicherte Desorbat als Bestandteil der in Schritt (I-3) eingesetzten sauren wässrigen Lösung gemeinsam mit einer Säure ausgewählt aus der Gruppe bestehend aus Salzsäure, Schwefelsäure, Phosphorsäure und Mischungen derselben verwendet wird.

4. Verfahren gemäß Anspruch 3, bei welchem das in Schritt (III) erhaltene, an Aminobenzoesäure angereicherte Desorbat als Bestandteil der in Schritt (I-3) eingesetzten sauren wässrigen Lösung gemeinsam mit Salzsäure einer Konzentration von 15 Massen-% bis 37 Massen-% verwendet wird.

5. Verfahren gemäß einem der vorstehenden Ansprüche, bei welchem der pH-Wert des wässrigen Desorptionsmittels während der Durchführung des Schrittes (III) konstant gehalten wird.

6. Verfahren gemäß einem der Ansprüche 1 bis 4, bei welchem der pH-Wert des wässrigen Desorptionsmittels während der Durchführung des Schrittes (III) einen Gradienten durchläuft.

7. Verfahren gemäß einem der vorstehenden Ansprüche, bei welchem in Schritt (I) eine wässrige Lösung des ortho-Isomers von Aminobenzoat und/oder von Aminobenzoesäure bereitgestellt wird.

8. Verfahren gemäß einem der vorstehenden Ansprüche, bei welchem die in Schritt (I-1) eingesetzten Mikroorganismen eine Art ausgewählt aus der Gruppe bestehend aus *Escherichia coli, Pseudomonas putida, Corynebacterium glutamicum, Ashbya gossypii, Pichia pastoris, Hansenula polymorpha, Yarrowia lipolytica, Zygosaccharomyces bailii und Saccharomyces cerevisiae,* umfassen und bevorzugt nur aus Vertretern genau einer dieser Arten bestehen, wobei *Corynebacterium glutamicum* ATTC 13032 ganz besonders bevorzugt ist.

## Claims

1. Process for preparing aminobenzoic acid or an aminobenzoic acid conversion product, comprising the steps of:
(I) providing an aqueous solution of aminobenzoic acid using a fermentation process, step (I) comprising:
(I-1) fermenting a raw material comprising at least
• a fermentable carbon-containing compound, preferably selected from the group consisting of starch hydrolysate, sugar cane juice, sugar beet juice and hydrolysates of lignocellulose-containing raw materials,
and
• a nitrogen-containing compound, preferably selected from the group consisting of ammonia gas, ammonia water, ammonium salts (especially ammonium sulfate and ammonium chloride) and urea,
in a fermentation reactor using microorganisms to obtain a fermentation broth; optionally followed by
(I-2) a work-up comprising the following work-up steps:
(α) removing the microorganism from the fermentation broth obtained in step (I-1) and/or
(β) decolorizing the fermentation broth obtained in step (I-1) or, when carrying out step (α), the microorganism-depleted fermentation broth obtained in step (α),
(1-3) introducing the fermentation broth obtained in step (I-1) or, if carried out, in step (I-2) into a reactor in which aminobenzoic acid is precipitated by mixing with an acidic aqueous solution, the pH of the resulting mixture being preferably adjusted to a value in the range from 3.0 to 4.7, preferably in the range from 3.2 to 3.7 and particularly preferably in the range from 3.4 to 3.6;
(1-4) removing the aminobenzoic acid precipitated in step (1-3), preferably by filtration, to obtain an aminobenzoic acid-depleted mother liquor;
(II) treating the aqueous solution of aminobenzoic acid provided in step (I), namely the aminobenzoic acid-depleted mother liquor obtained in step (1-4), with an adsorbent, preferably activated carbon, to load the adsorbent with aminobenzoic acid and to obtain an aminobenzoic acid-depleted adsorbate;
(III) treating the aminobenzoic acid-loaded adsorbent from step (II) with an aqueous desorbent of a pH in the range from -0.8 to 3.0, preferably -0.5 to 3.0, particularly preferably 0.1 to 3.0, very particularly preferably 0.5 to 2.5 and extremely very particularly preferably 1.0 to 2.0, to obtain an aminobenzoic acid-enriched desorbate and an aminobenzoic acid-depleted adsorbent;
(IV) obtaining the aminobenzoic acid from the desorbate obtained in step (III) by using the aminobenzoic acid-enriched desorbate obtained in step (III) as constituent of the acidic aqueous solution used in step (1-3);
(V) optionally further reacting the aminobenzoic acid obtained in step (IV) to give an aminobenzoic acid conversion product, step (V) comprising in particular one of the following reactions:
(V-1) decarboxylating the aminobenzoic acid to give aniline;
(V-2) decarboxylating the aminobenzoic acid to give aniline, followed by acid-catalysed reaction of the aniline with formaldehyde to form di- and polyamines of the diphenylmethane series;
(V-3) decarboxylating the aminobenzoic acid to give aniline, followed by acid-catalysed reaction of the aniline with formaldehyde to form di- and polyamines of the diphenylmethane series, followed by reaction with phosgene to form di- and polyisocyanates of the diphenylmethane series;
(V-4) decarboxylating the aminobenzoic acid to give aniline, followed by reaction of the aniline to give an azo compound;
(V-5) reacting the aminobenzoic acid to give an amide;
(V-6) reacting the aminobenzoic acid to give conductive polymers such as, in particular, polyanthranilic acid.

2. Process according to Claim 1, in which step (I) further comprises:
(1-5) further purifying the aminobenzoic acid removed in step (1-4), preferably by washing with water.

3. Process according to Claim 1 or 2, in which the aminobenzoic acid-enriched desorbate obtained in step (III) is used as constituent of the acidic aqueous solution used in step (I-3) together with an acid selected from the group consisting of hydrochloric acid, sulfuric acid, phosphoric acid and mixtures thereof.

4. Process according to Claim 3, in which the aminobenzoic acid-enriched desorbate obtained in step (III) is used as constituent of the acidic aqueous solution used in step (I-3) together with hydrochloric acid of a concentration of from 15% by mass to 37% by mass.

5. Process according to any of the preceding claims, in which the pH of the aqueous desorbent is kept constant while step (III) is carried out.

6. Process according to any of Claims 1 to 4, in which the pH of the aqueous desorbent passes through a gradient while step (III) is carried out.

7. Process according to any of the preceding claims, in which an aqueous solution of the ortho-isomer of aminobenzoate and/or of aminobenzoic acid is provided in step (I).

8. Process according to any of the preceding claims, in which the microorganisms used in step (I-1) comprise a species selected from the group consisting of *Escherichia coli, Pseudomonas putida, Corynebacterium glutamicum, Ashbya gossypii, Pichia pastoris, Hansenula polymorpha, Yarrowia lipolytica, Zygosaccharomyces bailii* and *Saccharomyces cerevisiae* and preferably only consist of representatives of precisely one of said species, *Corynebacterium glutamicum* ATTC 13032 being very particularly preferred.

## Revendications

1. Procédé de préparation de l'acide aminobenzoïque ou d'un dérivé de l'acide aminobenzoïque, comprenant les étapes :
(I) fourniture d'une solution aqueuse d'acide aminobenzoïque par utilisation d'un procédé fermentatif, l'étape (I) comprenant :
(I-1) la fermentation d'une matière première qui comprend au moins
• un composé carboné fermentable, de préférence choisi dans le groupe consistant en l'hydrolysat d'amidon, le jus de canne à sucre, le jus de betterave à sucre et les hydrolysats de matières premières lignocellulosiques, et
• un composé azoté, de préférence choisi dans le groupe consistant en l'ammoniac gazeux, l'ammoniaque, les sels d'ammonium (en particulier le sulfate d'ammonium et le chlorure d'ammonium) et l'urée,
dans un réacteur de fermentation par utilisation de microorganismes avec obtention d'un bouillon de fermentation ; puis, éventuellement,
(I-2) un traitement comprenant les étapes de traitement suivantes :
(α) séparation du microorganisme du bouillon de fermentation obtenu dans l'étape (I-1),
et/ou
(β) décoloration du bouillon de fermentation obtenu dans l'étape (I-1) ou, lors de la mise en œuvre de l'étape (α), du bouillon de fermentation obtenu dans l'étape (α), appauvri en microorganismes,
(I-3) introduction du bouillon de fermentation obtenu dans l'étape (I-1) ou, si elle est mise en œuvre, dans l'étape (I-2), dans un réacteur dans lequel l'acide aminobenzoïque est déposé par mélange avec une solution aqueuse acide, le pH du mélange obtenu étant alors de préférence ajusté dans la plage de 3,0 à 4,7, de préférence dans la plage de 3,2 à 3,7, d'une manière particulièrement préférée dans la plage de 3,4 à 3,6 ;
(I-4) séparation de l'acide aminobenzoïque déposé dans l'étape (I-3), de préférence par filtration, avec obtention d'une lessive mère appauvrie en acide aminobenzoïque ;
(II) traitement de la solution aqueuse d'acide aminobenzoïque fournie dans l'étape (I), plus précisément de la lessive mère obtenue dans l'étape (I-4), appauvrie en acide aminobenzoïque, avec un adsorbant, de préférence du charbon actif, ce à l'occasion de quoi l'adsorbant est chargé d'acide aminobenzoïque, et on obtient un adsorbat appauvri en acide aminobenzoïque ;
(III) traitement de l'adsorbant chargé en acide aminobenzoïque de l'étape (II) avec un désorbant aqueux ayant un pH dans la plage de -0,8 à 3,0, de préférence de -0,5 à 3,0, d'une manière particulièrement préférée de 0,1 à 3,0, d'une manière tout particulièrement préférée de 0,5 à 2,5, d'une manière exceptionnellement tout particulièrement préférée de 1,0 à 2,0, avec obtention d'un désorbat enrichi en acide aminobenzoïque et d'un adsorbant appauvri en acide aminobenzoïque ;
(IV) préparation de l'acide aminobenzoïque à partir du désorbat obtenu dans l'étape (III) par utilisation du désorbat obtenu dans l'étape (III), enrichi en acide aminobenzoïque, en tant que constituant de la solution aqueuse acide utilisée dans l'étape (I-3) ;
(V) éventuellement, réaction plus poussée de l'acide aminobenzoïque préparé dans l'étape (IV) pour donner un dérivé de l'acide aminobenzoïque, l'étape (V) comprenant en particulier l'une des réactions suivantes :
(V-1) décarboxylation de l'acide aminobenzoïque en aniline ;
(V-2) décarboxylation de l'acide aminobenzoïque en aniline, suivie d'une réaction catalysée par un catalyseur acide de l'aniline avec du formaldéhyde avec formation de di- et de polyamines de la série du diphénylméthane ;
(V-3) décarboxylation de l'acide aminobenzoïque en aniline, suivie d'une réaction catalysée par un catalyseur acide de l'aniline avec du formaldéhyde avec formation de di- et de polyamines de la série du diphénylméthane, suivie d'une réaction avec le phosgène avec formation de di- et de polyisocyanates de la série du diphénylméthane ;
(V-4) décarboxylation de l'acide aminobenzoïque en aniline, suivie d'une réaction de l'aniline conduisant à un composé azoïque ;
(V-5) réaction de l'acide aminobenzoïque conduisant à un amide ;
(V-6) réaction de l'acide aminobenzoïque conduisant à des polymères conducteurs tels en particulier que le poly(acide anthranilique).

2. Procédé selon la revendication 1, dans lequel l'étape (I) comprend en outre :
(I-5) une purification plus poussée de l'acide aminobenzoïque séparé dans l'étape (I-4), de préférence par lavage à l'eau.

3. Procédé selon la revendication 1 ou 2, dans lequel le désorbat obtenu dans l'étape (III), enrichi en acide aminobenzoïque, est utilisé en tant que constituant de la solution aqueuse acide utilisée dans l'étape (I-3) conjointement avec un acide choisi dans le groupe consistant en l'acide chlorhydrique, l'acide sulfurique, l'acide phosphorique et les mélanges de ceux-ci.

4. Procédé selon la revendication 3, dans lequel le désorbat obtenu dans l'étape (III), enrichi en acide aminobenzoïque, est utilisé en tant que constituant de la solution aqueuse acide utilisée dans l'étape (I-3) conjointement avec de l'acide chlorhydrique à une concentration de 15 % en masse à 37 % en masse.

5. Procédé selon l'une des revendications précédentes, dans lequel le pH du désorbant aqueux est maintenu constant pendant la mise en œuvre de l'étape (III) .

6. Procédé selon l'une des revendications 1 à 4, dans lequel le pH du désorbant aqueux passe par un gradient pendant la mise en œuvre de l'étape (III).

7. Procédé selon l'une des revendications précédentes, dans lequel, dans l'étape (I), on prépare une solution aqueuse de l'isomère ortho d'un aminobenzoate et/ou de l'acide aminobenzoïque.

8. Procédé selon l'une des revendications précédentes, dans lequel les microorganismes utilisés dans l'étape (I-1) comprennent une espèce choisie dans le groupe consistant en *Escherichia coli, Pseudomonas putida, Corynebacterium glutamicum, Ashbya gossypii, Pichia pastoris, Hansenula polymorpha, Yarrowia lipolytica, Zygosaccharomyces bailii et Saccharomyces cerevisiae,* et ne sont de préférence constitués que de représentants d'exactement l'une de ces espèces, *Corynebacterium glutamicum* ATTC 13032 étant tout particulièrement préféré.
